# EUROPEAN PATENT APPLICATION

(11) **EP 2 689 679 A1**
(43) Date of publication of application: **29.01.2014**
(21) Application number: 12178139.7
(22) Date of filing: 26.07.2012
(51) Int. Cl.: A41G 1/00, A61L 9/12

(54) **Dispensing device for a volatile substance**

(71) Applicant: Acuros GmbH, 13357 Berlin (DE)
(72) Inventor: Adleff, Helge, 10407 Berlin (DE)
(74) Representative: Zimmermann & Partner

(57) **Abstract**

A dispensing device for a volatile substance includes a diffuser (10) including a diffuser body; a storing reservoir (5) in fluid communication with the diffuser (10) and configured to store a liquid (6) containing at least one volatile substance; an osmotic pump (1) including an osmotically active substance (3) in contact with a first surface of a semipermeable membrane (2) of the osmotic pump (1), wherein the osmotically active substance is capable of forming a driving fluid (4) to act on the liquid (6) in the storing reservoir (5) and to drive the liquid (6) to the diffuser (10), when a second surface of the semipermeable membrane (2) is brought in contact with a solvent.

## Description

### FIELD OF THE INVENTION

The present invention relates to volatile substances dispensing devices. In particular it relates to dispensing devices for volatile substances having an osmotic pump for delivering said substances to a diffuser.

### BACKGROUND OF THE INVENTION

Dispensing of volatile substances is subject to many applications like perfuming, air freshening, room scenting, deodorizing, repelling or attracting insects and other animals, and also for medical applications like the treatment of respiratory diseases. Many different dispensing systems and methods are known in the relevant arts, including passive devices with wicks or other evaporation surfaces, devices using capillary forces to transport the volatile liquids to such evaporation surfaces or diffusers, manual or electrical or pressurized aerosol spray devices, active devices with electrical heaters to promote evaporation or diffusion, devices with electrical pumps or fans.

Active transport of volatile substances from a reservoir to a place of emission, diffusion or vaporization is used in many devices for control of the dispensing rate. Some known dispensing devices are designed like artificial flowers.

International patent application WO 2010/076003 discloses an artificial flower comprising a reservoir for a scent and a conveying apparatus for the scent. US patent application 2010/0044458 discloses an artistic display combining visual and olfactory appealing elements.

All of these volatile substances dispensing devices rely at least on one out of electricity, pressure significantly above ambient or manual activity.

### SUMMARY OF THE INVENTION

According to an aspect of the invention, a dispensing device according to claim 1 is provided. According to a further aspect, an artificial flower according to claim 11 is provided. According yet another embodiment, a method for dispensing a volatile substance according to claim 12 is provided. According to yet a further embodiment, use of a dispensing device to dispense a volatile substance according to claim 15 is provided.

According to an embodiment, a dispensing device for a volatile substance is provided which includes a diffuser having a diffuser body; a storing reservoir in fluid communication with the diffuser and configured to store a liquid containing at least one volatile substance; and an osmotic pump having an osmotically active substance in contact with a first surface of a semipermeable membrane of the osmotic pump. The osmotically active substance is capable of forming a driving fluid to act on the liquid in the storing reservoir and to drive the liquid to the diffuser, when a second surface of the semipermeable membrane is brought in contact with a solvent.

The dispensing device is activated by contacting or wetting the semipermeable membrane with the solvent such as water. The imbibed solvent drives the liquid to the dispenser where it evaporates. The acting of the osmotic pump can be stopped by discontacting the semipermeable membrane from the solvent, for example by removing the osmotic pump from a solvent reservoir. The pump will also stop when the solvent is consumed. The dispensing device is easy to use and does not need electricity or any other form of externally delivered energy.

According to an embodiment, the liquid includes a solvent in which the volatile substance is dissolved. The solvent may also be volatile and evaporate from the diffuser. According to an embodiment, the liquid is comprised of a volatile substance so that no additional solvent is needed.

According to an embodiment, the first surface of the semipermeable membrane forms an inner surface of the membrane facing to an interior of the osmotic pump while the second surface of semipermeable membrane forms an outer surface of the membrane exposed to the outside.

According to an embodiment the dispensing device includes an impermeable member separating the osmotically active substance and/or the driving fluid from the liquid, wherein the impermeable member is adapted to transmit the pressure generated by the driving fluid on the liquid.

The impermeable member can be, according to an embodiment, a foil or a flexible membrane which separates the osmotically active substance and/or the driving fluid from the liquid of the storing reservoir. According to an embodiment, the impermeable membrane can form a part of the reservoir or forms the reservoir. An example is a balloon containing the liquid.

According to an embodiment the dispensing device further includes a solvent reservoir and/or an absorbing substrate for absorbing the solvent, wherein the solvent reservoir and/or the absorbing substrate is/are in contact with the second surface of the semipermeable membrane. The absorbing substrate absorbes or stores the solvent, such as water, and is adapted to release the solvent so that the solvent can be imbibed by the osmotic pump.

According to an embodiment, the diffuser body includes a plurality of capillaries or a network of open pores extending to an outer surface of the diffuser body. This facilitates dispensing the volatile substance over a large surface to improve evaporation. According to an evaporation, the diffuser body is adapted to allow the volatile substance to evaporate faster than volatile substance is delivered to the diffuser. This ensures that no volatile substance drops off the diffuser.

According to an embodiment, the diffuser body includes at least one material selected from the group consisting of: a paper, a membrane, a sponge, a leather, a fabric, a gel, a ceramic, a powder, a cake, a crust, a packed mass, a wooden structure, and a dry part or remnant of a plant or animal.

According to an embodiment, an outer contour of the dispensing device has the appearance of a flower with the diffuser body forming at least a part of the bloom of the flower.

According to an embodiment, the liquid contains an essential oil, a fragrance, a solution comprising a solvent and at least one volatile substance, and mixtures thereof.

According to an embodiment, the liquid further contains a non-volatile substance such as a dye.

According to an embodiment, the liquid further contains at least two different non-volatile substances such as two different dyes and wherein the diffuser body forms a stationary phase for the two non-volatile dyes which, when pumped to the diffuser body, are chromatographically separated by the diffuser body. Upon delivery to the diffuser and subsequent evaporation of the volatile substance, the dyes are accumulated in the diffuser body and change the color of the diffuser body. Separate dyes having different mobility are chromatografically separated by the diffuser so that two- or multicoloring of the diffuser occurs.

According to an embodiment, an artificial flower includes a bloom and a support for the bloom. The bloom includes a diffuser having a diffuser body. The artificial flower further includes a storing reservoir in fluid communication with the diffuser and configured to store a liquid containing at least one volatile substance, wherein the storing reservoir is arranged in at least one of the bloom and the support; an osmotic pump arranged in the support and having an osmotically active substance in contact with a first surface of a semipermeable membrane of the osmotic pump, wherein the osmotically active substance is capable of forming a driving fluid to act on the liquid in the storing reservoir and to drive the liquid to the diffuser, when a second surface of the semipermeable membrane is brought in contact with a solvent.

According to an embodiment, a method of dispensing a volatile substance includes :
- providing a dispensing device, wherein the reservoir of the dispensing device contains a liquid comprising at least one volatile substance;
- wetting the semipermeable membrane of the osmotic pump of the dispensing device with a solvent to imbibe the solvent by the osmotically active substance so that a driving fluid, acting on the liquid is formed, to pump the liquid contained in the reservoir to the diffuser where the volatile substance evaporates.

According to an embodiment, the liquid further includes at least one non-volatile dye which accumulates in the diffuser body while the volatile substance evaporates.

According to an embodiment, the method further includes:
- chromatographically separating different components of a dye or different dyes from each other by the diffuser body when the liquid is pumped to the diffuser.

According to an embodiment, a dispensing device is used to dispense a volatile substance.

The volatile substances dispensing device in accordance with the present invention provides an osmotic pump to transport a volatile liquid substance from a container or reservoir to a place of emission, diffusion or vaporization. The pump is activated by wetting a semipermeable membrane and delivers the volatile liquid in a substantially constant flow as long as the membrane is held wet. The device may be decorative or attractively designed, for example with a flower like appearance.

### BRIEF DESCRIPTION OF THE DRAWINGS

Figure 1 shows a volatile substances dispensing device designed like an artificial flower. An osmotic pump is incorporated in the flower stem. The flower-shaped device is placed in a vase which is filled with water.

Figure 2 shows an embodiment wherein the artificial flower like device is planted in a flower pot.

Figure 3 shows a schematic of an osmotic pump.

### DETAILED DESCRIPTION OF THE INVENTION

It is to be understood that this invention is not limited to the particular structures, process steps, applications or materials disclosed herein, but is extended to equivalents thereof as would be recognized by those ordinarily skilled in the relevant arts. It should also be understood that terminology employed herein is used for the purpose of describing particular embodiments only and is not intended to be limiting. The invention will now be described in detail with reference to a few exemplary embodiments, as illustrated in accompanying drawings. In the following description, numerous specific details are set forth in order to provide a thorough understanding of the invention. However, it will be apparent to one skilled in the art that the invention may be practiced without some or all of these specific details.

The volatile substances dispensing device in accordance with an embodiment of the present invention comprises an osmotic pump **1** which includes a semipermeable membrane **2.** The osmotic pump **1** stores an osmotically active substance **3** which is in contact with a first surface, which forms here an inner surface, of the semipermeable membrane **2.** A second surface, which forms here an outer surface, of the semipermeable membrane **2** is or can be brought in contact to water **14** or a wet or wettable material **12** forming an absorbing substrate. The osmotic pump **1** is activated by wetting the second or outer surface of the semipermeable membrane **2.** Due to the osmotic pressure caused by the osmotically active substance **3,** water is taken up by the osmotic pump **1** to from a driving fluid **4** by diluting or dissolving the osmotically active substance **3.** In case the osmotically active substance **3** is a swellable material, e.g. a swellable hydrogel, the substance **3** is building up pressure by swelling rather then forming a driving fluid. The driving fluid **4** or the swelling substance is acting on a liquid **6** or a reservoir **5** storing a liquid **6** containing at least one volatile substance and driving the liquid **6** through an opening **17** of the reservoir **5.** Once activated, the osmotic pump **1** delivers the liquid **6** containing the at least one volatile substance at a low and substantially constant rate as long as the outer surface of the semipermeable membrane **2** is wet or disposed to a humid environment.

In an embodiment, the delivery rate is between 10 and 50 µl/hr. The reservoir **5** can be designed to store enough liquid **6** for many days of continuous delivery. Preferably, the reservoir holds from 5 ml to 100 ml of the liquid **6** containing the at least one volatile substance.

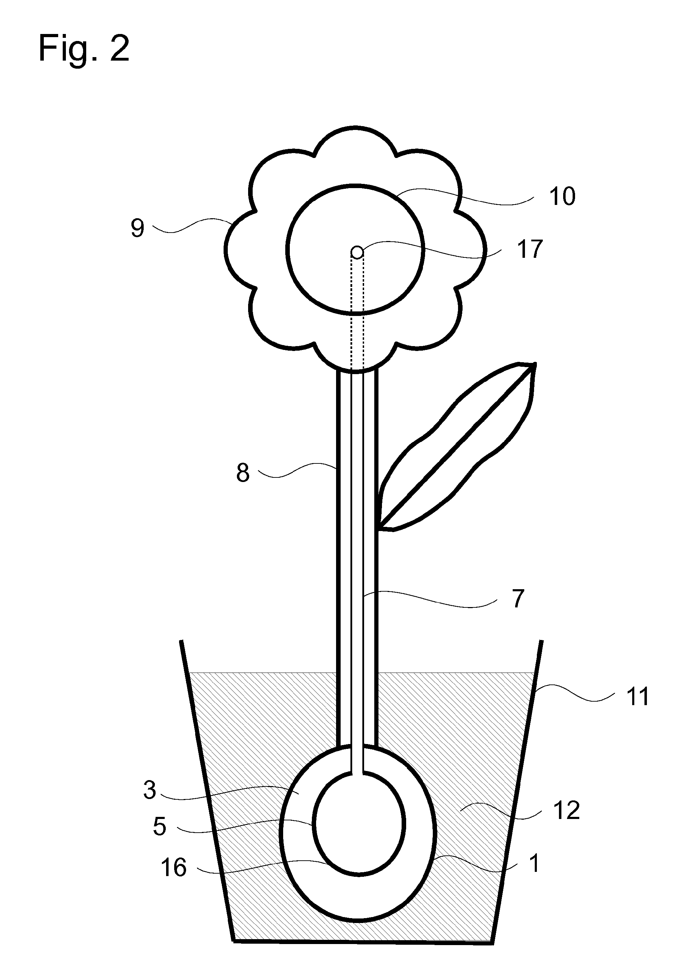

In an embodiment, the opening **17** is placed at an evaporation surface or into a diffuser **10** which is taking up the liquid **6** delivered by the osmotic pump **1.** The opening **17** can be formed as the end of a channel or tubing **7,** as shown in Figure 2, which is in fluid communication with reservoir **5.** The diffuser **10** is providing a surface large enough to evaporate the liquid **6** at a rate equal or higher than the delivery rate of pump **1.**

The volatile substances dispensing device according to an embodiment of the present invention can be appealingly designed, preferably it can have the appearance of an artificial flower. The diffuser **10** can be formed as the bloom or a part of it, e.g. petals, stamina or the calyx. The osmotic pump **1** can be formed like a bulb, a root tuber or can be integrated in an artificial flower stem **8** forming a support for the bloom **9.**

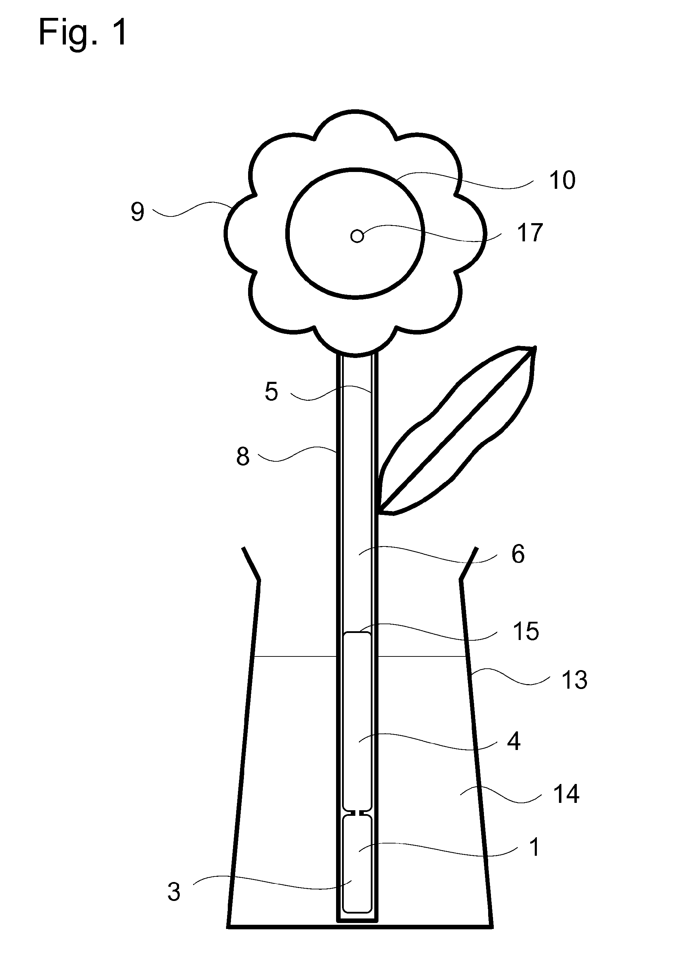

An exemplary embodiment of the volatile substances dispensing device is illustrated in Figure 1. The device is appealingly designed like an artificial flower. An osmotic pump **1** is integrated in the artificial flower stem **8.** The same flower stem **8** includes a reservoir **5** for a liquid **6** with at least one dissolved volatile substance. Alternatively such a reservoir **5** can be formed by a pouch, a plastic bag, a syringe, a tube, a balloon or other means integrated in the artificial flower stem **8** to store the liquid **6.** The reservoir **5** is connected to a diffuser **10** by an opening **17.** The opening **17** can be closed by default and opened manually, by pressure generated from the osmotic pump or by other means when the device is active. In Figure 1 the diffuser **10** is forming the receptacle of the artificial bloom **9,** but it could as well be forming any other part of the flower, e.g. stamina, petals or leafs. The artificial flower of Figure 1 can be placed in a water **14** filled vase **13.** Here, the lower part of the artificial flower stem **8** is permeable for water and the semipermeable membrane of osmotic pump **1** is thereby wetted and activated. The pump 1 is osmotically imbibing water and produces a driving fluid **4** which, as illustrated in Figure 1, is driving the liquid **6** out of the reservoir through opening **17** towards diffuser **10.** An impermeable member like a plunger or a foil can be used to separate the liquid **6** comprising the at least one volatile substance from driving fluid **4.** Such a member is dispensable when the two liquids **4** and **6** are immiscible, e.g. the liquid **6** is an oily liquid while driving fluid **4** is water-based so that the two liquids do not mix as illustrated by the visible meniscus **15** in Figure 1. The osmotic pump **1** is designed to deliver a liquid at a substantially constant rate. Diffuser **10** is wettable by volatile liquid **6** and is designed to expose a surface large enough to evaporate at least the same rate of liquid **6** that is delivered by osmotic pump **1** in order to avoid liquid volatile substance **6** to drip from diffuser **10.**

Figure 2 illustrates another embodiment of the disclosed volatile substances dispensing device. This flower-shaped device is potted in a flower pot **11** together with a wettable substrate **12** forming an absorbing substrate. The osmotic pump **1** is activated by watering the wettable substrate **12.** The wettable substrate **12** can be designed to hold a limited amount of water in order to limit the amount of volatile liquid **6** dispensed by the device. This can be useful when dispensing is desired for e.g. a few hours daily. The device can then be activated many times by watering the wettable substrate **12** without consumption of liquid **6** when not required in the meantime. For example, the active time of an insect repellant dispensing device can be limited to some hours every time after activation. The osmotic pump **1** of the illustrated embodiment is designed to be an artificial bulb comprising a reservoir **5** for the volatile liquid **6.** Here, the reservoir **5** is formed by a compressible plastic pouch or balloon connected to a diffuser **10** by a tubing **7** with an opening **17.** The envelope 16 of the balloon forms a flexible or elastic impermeable member separating the osmotically active substance **3** from the liquid **6.**

Many different types of osmotic pumps are known and most of them can be used as an osmotic pump **1** in the volatile substances dispensing device disclosed in the present application. In an embodiment the osmotic pump **1** comprises a water reservoir. The pump is activated by bringing water from this water reservoir into contact with the semipermeable membrane. Hence, in this embodiment it is not necessary to dispose the pump into water or into a wet or wettable substrate to activate the delivery process. Such an osmotic pump comprising a water reservoir is disclosed by Adleff et al. in US patent application 2009/0129945 A1, the entire content of which is incorporated herein by reference. A dispensing device using this pump is not only independent from water supply but can also feature interrupted flow and different selectable flow rates.

In another exemplary embodiment the osmotic pump **1** is an osmotic pump according to US patent No. 7,976,535 by Ehwald et al., the entire content of which is incorporated herein by reference This osmotic pump uses a stationary osmotic gradient along the convective flow path in a dilution chamber. Hence, it is possible to move a volume of liquid **6** which is large in comparison to the volume of the osmotic pump **1** what is of advantage when small dispensing devices or long uptime is required.

In an exemplary embodiment both, the inner and outer surface of the semipermeable membrane **2** is wetted by the osmotically active substance **3** prior to use. In this state, the osmotic pressure difference across the membrane **2** is zero and the pump **1** is inactive. The device can be stored without loss of liquid substances. The osmotically active substance **3** is removed from the outer membrane surface prior to use of the device. In a more specific exemplary embodiment the osmotic pump **1** is integrated in the lower end of an artificial flower stem **8** and this end is disposed in a tube which was filled with a liquid of the same osmotic pressure of osmotically active substance **3.** The artificial stem **8** sticks through a seal or plug which holds the liquid in the tube. Prior to use the stem is pulled out of the tube, eventually rinsed with water to remove residuals of osmotic substances and then disposed in a vase filled with water or potted in a wettable substrate.

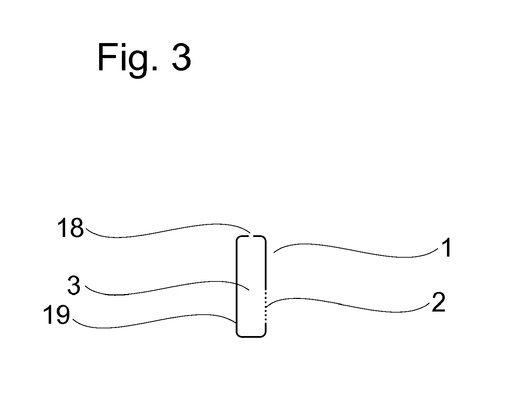

According to an embodiment, the semipermeable membrane **2** forms a part of an outer surface of the osmotic pump **1,** for example it forms a part of a housing **19,** as shown in Figure 3. Presenting the semipermeable membrane **2** to the outside facilitates contacting the membrane **2** with the solvent such as water. As described above, the pump **1** can be simply placed into a water reservoir to activate the pump. This is different to osmotic pumps having the semipermeable membrane arranged within an outer housing of the pump.

The osmotic pump **1** includes a housing **19** which stores the osmotically active substance **3.** The housing has an opening **18** through which the osmotically active substance **3** or the driving fluid 4 formed by uptake of the solvent is pumped when the solvent such as water is imbibed through the semipermeable membrane **2.** The partially diluted osmotically active substance **3** forms than a driving fluid **4** which acts either directly on the liquid **6,** as shown in Figure 1, or indirectly through the impermeable member **16,** as shown in Figure 2. The semipermeable membrane **2** can by supported by a net, which can be integrally formed with the housing **19** or can simply cover holes formed in the housing **19.** Opening **18** can be covered by suitable means to allow connection with tubes or other fluid conducting elements to guide the driving fluid **4** to the reservoir **5** or to the liquid **6.**

In another exemplary embodiment the diffuser **10** is made from a material wettable by volatile liquid **6** but repellant to driving fluid **4,** or the diffuser **10** is separated from the opening **17** by such a material. For example, the volatile liquid **6** is an oily liquid and the diffuser **10** or the separating element is a porous membrane or a non-woven made from an oleophilic material, e.g. an olefin. Volatile liquid **6** uptake to such a diffuser is promoted whilst the diffuser will not be soaked with driving fluid **4** once all volatile liquid **6** is dispensed. Instead, the pressure inside the osmotic pump **1** rises and driving fluid **4** will only be dispensed when the break-through pressure of diffuser **10** or the separating element for the driving fluid **4** is exceeded. Leakage of driving fluid **4** through diffuser **10** can be prevented when an alternative flow path opens at a pressure below said break-through pressure. Such a flow path can be opened by a pressure sensitive valve, another membrane or porous material expressing a lower break-through pressure then diffuser **10** or other pressure relieve means known in the relevant arts. Leakage of driving fluid **4** through diffuser **10** can also be prevented by an impermeable barrier, e.g. a plunger or impermeable foil, which is disposed between the two liquids **4** and **6.** Such means applied, the pressure inside the pump rises when volatile liquid **6** is completely consumed whilst osmotically active substance **3** is still available and the outer surface of the semipermeable membrane **2** is wet or disposed in a humid environment and the rising pressure can be used to open alternative flow paths like described above.

In a further exemplary embodiment of the present invention the dispensed liquid **6** is a solution comprising at least a volatile substance and at least a non-volatile substance. In a more specific exemplary embodiment, the at least one non-volatile substance dissolved in volatile liquid **6** comprises at least one dye. In an active device the at least one dye accumulates in the diffuser **10** and displays the state of the device and can contribute to the appealing design of the dispensing device. Since the amount of the at least one dye arrived at the diffuser increases over time the appearance of the diffuser **10** changes and this may contribute to a vivid impression of e.g. an artificial flower. This impression can be further increased when a mixture of dyes comprising different colors is used. The diffuser **10** then acts like a stationary phase, wherein different dyes are separated chromatographically into different colors by the liquid **6** acting as a mobile phase. Advantageously this may cause an ever-changing appearance of the diffuser and is of special interest for the design of artificial petals.

In a preferred embodiment the osmotic pump **1** of a volatiles substances dispenser according to an embodiment of the present invention is exchangeable and the dispensing device is reusable. The reservoir **5** can also be exchangeable or refillable to be refilled with volatile liquid **6** containing at least one fragrance or at least one fragrance and at least one dye. Moreover, the diffuser **10** can also be made exchangeable.

## Claims

1. Dispensing device for a volatile substance, comprising:
- a diffuser (10) comprising a diffuser body;
- a storing reservoir (5) in fluid communication with the diffuser (10) and configured to store a liquid (6) comprising at least one volatile substance;
- an osmotic pump (1) comprising an osmotically active substance (3) in contact with a first surface of a semipermeable membrane (2) of the osmotic pump (1), the osmotically active substance is capable of forming a driving fluid (4) to act on the liquid (6) in the storing reservoir (5) and to drive the liquid (6) to the diffuser (10), when a second surface of the semipermeable membrane (2) is brought in contact with a solvent.

2. Dispensing device according to claim 1, further comprising an impermeable member (16) separating the osmotically active substance (3) and/or the driving fluid (4) from the liquid (6), wherein the impermeable member is adapted to transmit the pressure generated by the driving fluid on the liquid (6).

3. Dispensing device according to claim 2, wherein the impermeable member (16) is a foil or a flexible membrane separating the osmotically active substance (3) from the liquid (6) of the storing reservoir (5).

4. Dispensing device according to any one of the claims 1 to 3, further comprising a solvent reservoir and/or an absorbing substrate (12) for absorbing the solvent, wherein the solvent reservoir and/or the absorbing substrate (12) is/are in contact with the second surface of the semipermeable membrane (2).

5. Dispensing device according to any one of the claims 1 to 4, wherein the diffuser body comprises a plurality of capillaries or a network of open pores extending to an outer surface of the diffuser body.

6. Dispensing device according to any one of the claims 1 to 5, wherein the diffuser body comprises at least one material selected from the group consisting of:
a paper, a membrane , a sponge, a leather, a fabric, a gel, a ceramic, a powder, a cake,
a crust, a packed mass, a wooden structure, and a dry part or remnant of a plant or animal.

7. Dispensing device according to any one of the claims 1 to 6, wherein an outer contour of the dispensing device has the appearance of a flower with the diffuser body forming at least a part of the bloom of the flower.

8. Dispensing device according to any one of the claims 1 to 7, wherein the liquid comprises an essential oil, a fragrance, a solution comprising a solvent and at least one volatile substance, and mixtures thereof.

9. Dispensing device according to claim 8, wherein the liquid further comprises a non-volatile substance such as a dye.

10. Dispensing device according to claim 8, wherein the liquid further comprises at least two different non-volatile substances such as two different dyes and wherein the diffuser body forms a stationary phase for the two non-volatile dyes which, when pumped to the diffuser body, are chromatographically separated by the diffuser body.

11. Artificial flower, comprising:
- a bloom (9) and a support (8) for the bloom (9), the bloom (9) comprising a diffuser (10) comprising a diffuser body;
- a storing reservoir (5) in fluid communication with the diffuser (10) and configured to store a liquid (6) comprising at least one volatile substance, the storing reservoir (5) is arranged in at least one of the bloom (9) and the support (8);
- an osmotic pump (1) arranged in the support (8) and comprising an osmotically active substance (3) in contact with a first surface of a semipermeable membrane (2), the osmotically active substance (3) is capable of forming a driving fluid (4) to act on the liquid (6) in the storing reservoir (5) and to drive the liquid (6) to the diffuser (10), when a second surface of the semipermeable membrane (2) is brought in contact with a solvent.

12. Method of dispensing a volatile substance, comprising:
- providing a dispensing device according to any one of the claims 1 to 10, wherein the reservoir (5) of the dispensing device contains a liquid (6) comprising at least one volatile substance;
- wetting the semipermeable membrane (2) of the osmotic pump (1) of the dispensing device with a solvent to imbibe the solvent by the osmotically active substance (3) so that a driving fluid, acting on the liquid (6) is formed, to pump the liquid (6) contained in the reservoir (5) to the diffuser (10) where the volatile substance evaporates.

13. Method according to claim 12, wherein the liquid (6) further comprises at least one non-volatile dye which accumulates in the diffuser body while the volatile substance evaporates.

14. Method according to claims 13 or 14, further comprising:
- chromatographically separating different components of a dye or different dyes from each other by the diffuser body when the liquid is pumped to the diffuser.

15. Use of despensing device according to any one of the claims 1 to 10 or of the artificial flower according to claim 11 to dispense a volatile substance.
